# EUROPEAN PATENT SPECIFICATION

(11) **EP 2 086 502 B1**
(45) Date of publication and mention of the grant of the patent: **17.04.2013**
(21) Application number: 07864085.1
(22) Date of filing: 08.11.2007
(51) Int. Cl.: A61L 24/06, A61K 9/00, A61F 9/00, A61P 27/00

(54) **PUNCTAL PLUG COMPRISING A WATER-INSOLUBLE POLYMERIC MATRIX**
PUNKTUELLER VERSCHLUSS MIT EINER WASSERUNLÖSLICHEN POLYMERMATRIX
BOUCHON MÉATIQUE COMPRENANT UNE MATRICE POLYMÈRE INSOLUBLE DANS L'EAU

(30) Priority: 09.11.2006 US 857833 P
(43) Date of publication of application: 12.08.2009
(73) Proprietor: Alcon Research, Ltd., Fort Worth, TX 76134-2099 (US)
(72) Inventor: ASGHARIAN, Bahram, Arlington, TX 76016 (US); CHOWHAN, Masood A., Arlington, Texas 76016 (US)
(74) Representative: Hanna, Peter William Derek
(86) International application number: PCT/US2007/084018
(87) International publication number: WO 2008/060929

(56) References cited:
- WO-A-02/11783
- WO-A-94/05342
- WO-A-2006/031658
- WO-A-2007/115259
- WO-A-2007/115261
- US-A- 3 949 750
- PERSTORP: 'CAPA 3031, 3041, 3050, 3091 and 3201 Polycaprolactones', [Online] 06 January 2005, pages 1 - 8, XP055003964 Retrieved from the Internet: <URL:http://www.perstorppolyols.com/upload/ capa3031_070420.pdf> [retrieved on 2011-08-01]
- PERSTORP: 'CAPA 4101 and 4801', [Online] 01 June 2005, pages 1 - 8, XP055003967 Retrieved from the Internet: <URL:http://www.perstorppolyols.com/upload/ capa4101_050601.pdf> [retrieved on 2011-08-01]
- IGOR TIAGO ET AL: "Metabolic and Genetic Diversity of Mesophilic and Thermophilic Bacteria Isolated from Composted Municipal Sludge on Poly-[epsilon]-caprolactones", CURRENT MICROBIOLOGY, SPRINGER-VERLAG, NE, vol. 49, no. 6, 1 December 2004 (2004-12-01), pages 407-414, XP019365528, ISSN: 1432-0991
- SOLVAY: 'The CAPA Range of Products' PRODUCT BROCHURE, [Online] 28 October 2003, CHESHIRE, UK, pages 1 - 2, XP055019690 Retrieved from the Internet: <URL:http://www.prochem.ch/html/forum/Capa_ Range_of_Product.pdf> [retrieved on 2012-02-17]

## Description

### BACKGROUND OF THE INVENTION

### A. Field of the Invention

The present invention relates generally to the field of implantable ocular compositions. More particularly, it concerns paste implantable compositions that can be used to treat ocular diseases or conditions. In preferred aspects, the ocular disease or condition to be treated is keratoconjunctivitis sicca (i.e, dry eyes or dry eye syndrome).

### B. Background of the Invention

The tear film is a complex structure that serves to protect the surface of the eye. The tear film includes three basic layers: an outer lipid layer, an inner mucin layer, and an aqueous layer between the lipid and mucin layers. Each of the layers has a particular function. The lipid layer prevents evaporation of the tears from the surface of eye. The aqueous layer provides oxygen to the cornea and contains additional chemical components that are important to a healthy eye. The mucin layer provides for interaction between the lipid layer and the aqueous layer and prevents tears from "beading up" on the cornea.

Approximately 7.5 million cases of moderate or severe dry eye syndrome occur in the United States each year. An additional five million people are unable to wear contact lenses because of insufficient amounts of tear fluid. A "dry eye" is one that experiences insufficient lubrication of the cornea as a result of a disturbance in the normal tear film. The condition encompasses a wide variety of signs and symptoms ranging from mild, intermittent burning and/or scratchiness with foreign body sensation, to a severe lack of aqueous layer secretion accompanied by corneal and conjunctival disease (keratoconjunctivitis sicca (KCS)).

Dry eye can have a variety of specific causes and contributing factors, including arid environments, environmental airborne pollutants, certain systemic medications, auto-immune disorders, drug toxicity, hormone deficiency or changes, and even contact lens wear. However, the majority of cases of dry eye syndrome are related to two basic causes. First, the lacrimal duct from the lacrimal gland may become clogged or may malfunction so that an insufficient amount of tears reach the eye. In response to this cause, artificial tear products such as TEARS NATURALE™ and BION™ TEARS, sold by Alcon™ Laboratories, Inc. of Fort Worth, Tex., were developed. Second, although the lacrimal gland and lacrimal duct may deliver a sufficient amount of tears to the eye, tears may be drained away from the eye too quickly, creating a dry eye situation. In response, various methods and apparatus for sealing the puncta have been developed.

Initially, puncta were sealed by stitching or by electrical or laser cauterization. Although such procedures can provide acceptable results, they are not reversible without reconstructive surgery. As it is sometimes difficult to determine whether dry eye is caused by too great of drainage or too little tear production, such procedures may expose the patient to unnecessary trauma. In addition, such procedures may result in epiphora, a condition where tears continually form on the eye, build up, and run down the face of the patient.

Pre-formed collagen plugs for insertion into puncta or the canaliculi were developed to provide a reversible sealing procedure. Collagen plugs are water-soluble and, when inserted into the puncta, typically dissolve within seven to fourteen days. Collagen plugs are thus effective as a test procedure to determine if it is desirable to more permanently seal the puncta.

Pre-formed water-insoluble plugs for insertion into puncta or canaliculi are described in a variety of United States Patents. For example, U.S. Pat. No. 3,949,750 to Freeman describes such a plug having a head portion that extends outside of the punctum and a barb portion that extends into the punctum and/or canaliculus. Such plugs can be seen in the corner of the eye, are sometimes uncomfortable, and are easily dislodged. In addition, such plugs are somewhat difficult to insert, and occasionally their size and shape causes tissue damage during insertion. If such plugs protrude too far from the puncta, they can cause irritation to the sclera. Furthermore, the tissue of the punctum can be damaged due to prolonged dilation caused by such plugs.

U.S. Pat. No. 5,283,063 to Freeman describes a similar plug made from a hydrogel material having a hydrating port located in its barb portion that allows canalicular fluid to enter the barb and hydrate the plug to an expanded, relatively flexible state. U.S. Pat. Nos. 5,723,005 and 5,171,270 to Herrick describe water-insoluble punctal plugs that have collapsible flared sections for improved sealing and anchoring within the canaliculus. Some of these plugs also have a retaining portion that extends outside the punctum to further anchor the plug and prevent migration down the canaliculus.

U.S. Pat. No. 5,469,867 to Schmitt describes a method of occluding the lacrimal canaliculi and other mammalian channels or ducts by injecting a heated, flowable polymer or polymer composite of a specified composition through puncta into canaliculi, respectively. The specified polymer and polymer composite are non-immunogenic, biocompatible materials that are solid and/or non-flowable at body temperature and flowable when heated slightly above body temperature. The polymer and polymer composite are capable of quickly changing from a flowable state to a non-flowable state by moving through only a few centigrade degrees of temperature. After injection, the polymer or polymer composite cools and solidifies to form a plug that conforms exactly to the geometry of the canaliculi. However, the heated polymer and the heated delivery device have the potential to cause heat bums during insertion, which can result in damage to delicate tissue structures. Another concern is the melt temperature of the polymer. If the melt temperature is higher than the body temperature of the subject, there is a significant risk of heat bums to delicate tissue. Further, if the melt temperature is below 45° C, unwanted softening can occur, such as when a subject has a fever or sits in front of a fireplace. This can result in loss of the material from the lacrimal system.

### SUMMARY OF THE INVENTION

The present invention overcomes the deficiencies in the art by providing for a bio-compatible water-insoluble matrix that can be formulated into an injectible composition that can be used to occlude a channel in a subject.

More particularly, the present invention generally pertains to injectable paste compositions in accordance with claims which follow. Exemplary compositions include a water insoluble polymer matrix that includes a polyester polymer or a fatty acid based polymer, or a mixture of both polymers, wherein the polymer matrix has a melting point of less than 60°C, and wherein the composition is a paste at room temperature and is formulated to occlude a channel in a subject.

The term "subject" refers to either a human or non-human, such as primates, mammals, and vertebrates. In particular embodiments, the subject is a human.

A "channel is defined herein to refer to a tubular passage in a subject. In particular embodiments, the channel is a canalicular channel or punctal channel.

To "occlude" or "occlusion" as used in the context of the present invention refers to impeding or blocking passage through the channel relative to passage in the absence of the composition of the present invention. Thus, "occlude" as used herein refers to both total and partial obstruction of a channel.

The duration of the occlusion can be permanent or temporary. It can last for any period of time. For example, in particular embodiments, the composition is formulated to occlude a channel for at least one year. In other embodiments, the composition is formulated to occlude the channel for less than one year.

The composition can include any additional components known to those of ordinary skill in the art. For example, in some embodiments, the composition includes up to 20% of a water miscible organic liquid. The water miscible organic solvent may or may not be miscible with the polymer matrix. In particular embodiments, the water miscible organic liquid is miscible within the polymer matrix.

The polyester polymer is a polyester polymer selected from the group consisting of: poly(caprolactone)s; poly(ethylene glycol adipate)s; poly(propylene glycol adipate)s; poly(butylene glycol adipate)s; poly(hydroxybutarate)(s); poly(hydroxyvalerate)(s); and blends and copolymers thereof as defined in the claims. In particular embodiments, the polymer matrix comprises a poly(caprolactone) polymer as defined in the claims. The polymer matrix may include a polyester polymer selected from the group consisting of: 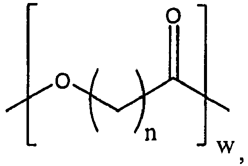 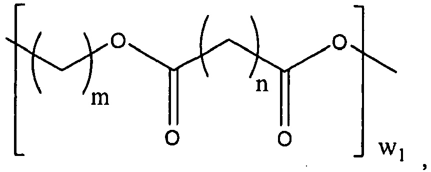 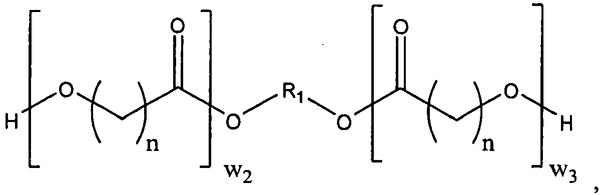 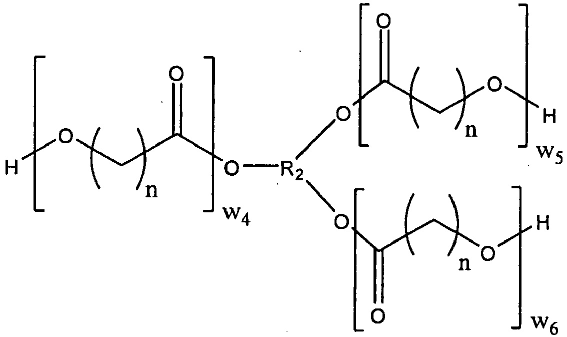 and 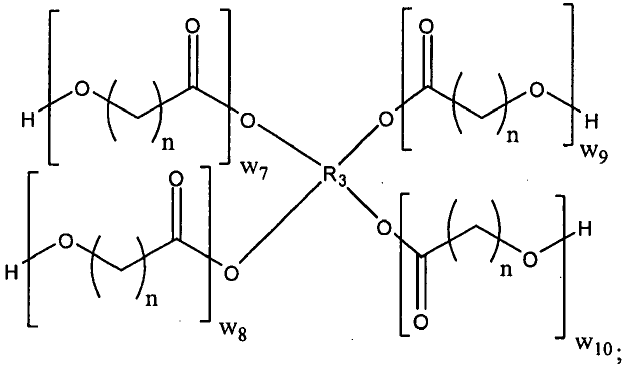 wherein
R₁, R₂ and R₃ are independently selected from the group consisting of alkyl and alkoxyl diols, triols and tetraols of 2 to 8 carbon atoms;
w, w₁ are independently an integer from 4 to 12;
w₂, w₃ are independently an integer from 1 to 12;
w₄, w₅, w₆, w₇, w₈, w₉ and w₁₀ are independently an integer from 0 to 12;
n is an integer from 4 to 9; and
m is an integer from 2 to 8.

Non-limiting examples of the alkyl and alkoxyl diols, triols and tetraols of R₁, R₂ and R₃ include butanediol, hexanediol, neopentyl glycol, diethylene glycol, trimethylol propane and pentaerythritol. An example of a polyester polyol wherein R₁ is butanediol is as follows:

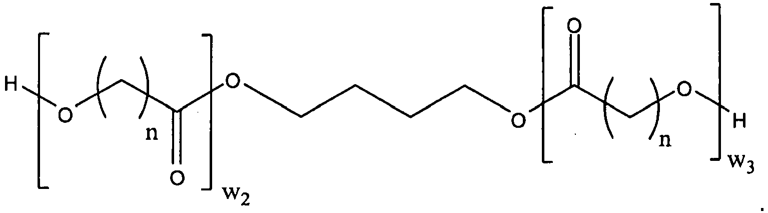

The fatty acid-based polymer of the pharmaceutical compositions of the present invention is a fatty acid based polymer having the following structure:

A-(B-A)ₙ-B-A,

wherein
A is an aliphatic hydroxycarboxylic acid ester from 6 to 60 carbon atoms;
B is a diacid dimer of unsaturated fatty acids from 8 to 40 carbon atoms; and
n is an integer from 0 to 3.

An "aliphatic hydroxycarboxylic acid ester" is defined herein to refer to a straight, branched and/or cyclic aliphatic hydrocarbon chain comprising one or more hydroxy groups and one or more ester groups. For example, in some embodiments, A has the structure: 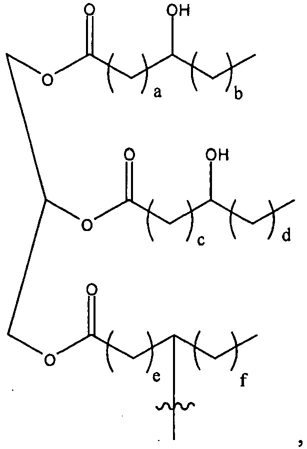 wherein a, b, c, d, e and f are independently alkyl groups ranging from 1-15 or more linear carbons.

A "diacid dimer" is defined herein to refer to a dimer comprising a cyclic aliphatic and/or aromatic hydrocarbon core structure of 5-8 carbons comprising at least two carboxylic acid substituents that are covalently bound to at least two aliphatic hydroxycarboxylic acid esters. The core structure may comprise additional substituents, such as H and/or alkyl groups of 1-15 carbons. In some embodiments, B has the structure: 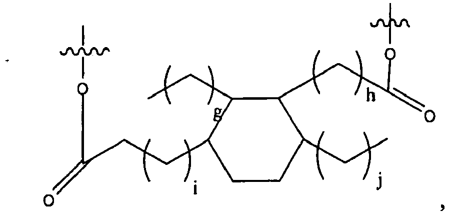 wherein g, h, i and j are independently alkyl groups ranging from 1-15 or more linear carbons.

The polymer matrix may contain polymers of any molecular weight. In some embodiments, the polymer matrix contains polymers having an average molecular weight of 400 to 8000. Further, the polymer matrix may be of any viscosity. In particular embodiments, the viscosity of the polymer matrix is from 50 to 8000 cps at 55 °C.

The polymer matrix may or may not be bioerodible. In particular embodiments, the polymer matrix or the composition is bioerodible.

In particular embodiments, one or more active agents is dispersed within the polymer matrix. Active agents include, but are not limited to, any component, compound, or small molecule that can be used to bring about a desired effect. For example, a desired effect can include the diagnosis, cure, mitigation, treatment, or prevention of a disease or condition. In particular embodiments, the active agent is a drug.

In some embodiments, the composition is formulated to controllably release the active agent for a pre-determined period of time. Controlled release formulations are well-known to those of ordinary skill in the art, and are discussed elsewhere in this specification.

The active agent can be any active agent known to those of ordinary skill in the art. For particular embodiments, the active agent is an ophthalmic drug. The active agent may or may not be in solution or suspension.

The composition can be formulated in any manner known to those of ordinary skill in the art. The composition is formulated into an injectible paste.

In some embodiments, the composition is comprised in a syringe or other device suitable for injection of the composition into a channel.

The present invention also generally pertains to the use of the composition for occluding a channel in a subject that involve administering one of the pharmaceutical compositions of the present invention into a channel of the subject. The composition may or may not require pre-heating prior to administration. In particular embodiments, the pharmaceutical composition is not pre-heated prior to administering the composition into the channel. As set forth above, the channel can be any channel. One example of a channel is a canalicular channel. Another example is a punctal channel.

The channel can be formulated to occlude the channel for any duration of time. In particular embodiments, the channel is occluded for at least one year. In other embodiments, the channel is occluded for shorter durations of time, such as one week, one month, and so forth.

Any method administering the composition known to those of ordinary skill in the art is contemplated by the present invention. For example, in some embodiments, the pharmaceutical composition is injected into the channel with a needle.

The pharmaceutical composition can be of any viscosity. In some embodiments, the pharmaceutical composition is non-flowable. In other embodiments, the pharmaceutical composition conforms to the shape of the channel.

The present invention also generally pertains to the composition for use in a method for treating dry eye syndrome in a subject that involve administering a pharmaceutical composition of the present invention into a canalicular or punctal channel of the subject, wherein the composition reduces drainage of tear fluid from the eye through the canalicular channel or punctal opening of the subject. In some embodiments, the pharmaceutical composition is not pre-heated prior to administering the composition into the canalicular or punctal channel.

The pharmaceutical composition that is administered to the subject may or may not be flowable. In some embodiments, the pharmaceutical composition forms to the shape of the canalicular or punctal channel. The pharmaceutical composition is formulated into an injectible paste.

The present invention also describes to kits that include a pharmaceutical composition of the present invention and a device for administering the pharmaceutical composition to a subject. Kits are discussed in greater detail in the specification below. The device for administering the pharmaceutical composition can be any device known to those of ordinary skill in the art. In the present invention the device is a syringe. It may also include a needle.

It is contemplated that any embodiment discussed in this specification can be implemented with respect to any method or composition of the invention, and *vice versa.* Furthermore, compositions of the invention can be used to achieve methods of the invention.

"Room temperature" includes the ambient temperature of a given room (*e.g.*, a lab, medicine cabinet, bathroom, *etc.),* and in most normal cases, this would encompass a temperature of about 15 °C to about 25 °C.

The phrases "occluding a channel" or "blocking a channel" or any variation of these phrases when used in the claims and/or the specification refers to a process of partially and/or completely filling at least a portion or section of a channel, passage, opening, cavity or space with a substance that hinders and/or completely prevents the transport or movement of another substance through the channel. This "other substance" could be biological in origin such as sperm, ova, tears or blood or it could be a prosthetic device such as a metal rod or pin. In preferred embodiments the channel is completely blocked and prevents all flow through.

The term "bioerodible" includes the degradation, disassembly, or digestion of the compositions and/or polymers of the present invention by action of a biological environmental cue (*e.g.*, acidity, temperature, or moisture of the target site, the existence of enzymes, proteins, or other molecules at the target site).

The term "matrix" pertains to the physical structure of the polymers of the present invention. In certain embodiments, an active agent can be incorporated with the polymer matrix.

The term "subject" refers to either a human or non-human, such as primates, mammals, and vertebrates. In particular embodiments, the subject is a human.

The term "about" or "approximately" are defined as being close to as understood by one of ordinary skill in the art, and in one non-limiting embodiment the terms are defined to be within 10%, preferably within 5%, more preferably within 1%, and most preferably within 0.5%.

The terms "inhibiting," "reducing," or "prevention," or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the word "a" or "an" when used in conjunction with the term "comprising" in the claims and/or the specification may mean "one," but it is also consistent with the meaning of "one or more," "at least one," and "one or more than one."

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description. It should be understood, however, that the detailed description and the examples, while indicating specific embodiments of the invention, are given by way of illustration only.

### BRIEF DESCRIPTION OF THE DRAWINGS

The following drawings form part of this specification and are included to further demonstrate certain non-limiting aspects of the present invention. The invention may be better understood by reference to one or more of these drawings in combination with the detailed description of specific embodiments presented in this specification.
**FIG. 1** is a cross sectional schematic view showing the lacrimal duct system of a mammalian eye;
**FIG. 2** is cross sectional schematic view of the canalicular channels blocked with the composition of the present invention.

### DESCRIPTION OF ILLUSTRATIVE EMBODIMENTS

Unless otherwise stated, all ingredient amounts presented as a percentage are in percent weight/weight terms (wt.%).

As indicated above, approximately 7.5 million cases of moderate or severe dry eye syndrome occur in the United States every year. The condition encompasses a wide variety of signs and symptoms ranging from mild, intermittent burning and/or scratchiness with foreign body sensation, to a severe lack of aqueous layer secretion accompanied by corneal and conjunctival disease (keratoconjunctivitis sicca (KCS)). Current treatment options are oftentimes ineffective and can be cumbersome. For instance, pre-formed water-insoluble plugs for insertion into puncta or canaliculi are sometimes uncomfortable, and are easily dislodged. Further, injectible compositions that require pre-heating can be cumbersome and can require specialized equipment.

The present invention overcomes these deficiencies in the art by providing a bio-compatible water-insoluble matrix that can be formulated into an injectible paste that can be used to occlude a channel in a subject. The water-insoluble matrix can include a bioerodable polyester polymer or a nonbioerodable fatty acid based polyester polymer, or a mixture thereof. In certain aspects, the composition can be used to treat dry-eye syndrome by administering the composition into the canalicular or punctal channels of a mammalian eye.

These and other non-limiting aspects of the invention are described in further detail in the following sections.

### A. Treatment Applications

In general, the compositions of the present invention can be used to block any type of channel (*e.g.*, block channels within living and non-living beings of any type and block all types of channels and tubular devices even outside of biological systems). The blocking of channels can prevent or reduce the movement of a substance into or out of a channel within that host using the compositions of the present invention.

Typically, the compositions of the present invention can be inserted into the channels by an injecting device (*e.g.*, any device capable of holding or containing the composition of the present invention in its chamber and injecting or extruding the composition from that container into the duct or channel to be blocked). Non-limiting examples of injecting devices that can be used include pointed plastic tip applicators, catheters, reservoirs, plungers, release systems, plastic tubes; fine cannula, tapered cannula and various types of syringes and hypodermic needles which are generally known to and available to those in the medical profession. The amount of the polymeric material provided in the injecting device can vary depending on the particular channel to be blocked and the amount and type of blockage desired. It is contemplated that those of ordinary skill in the art will understand that the amount of composition to be included within the injecting device can depend on the size of the channel being blocked. Additionally, the size of the tip or nozzle of the injecting device can be related to the inside diameter of the channel into which it is placed. For example, a 24 gauge needle easily fits within the opening of the punctum which leads to the canaliculus.

### 1. Dry Eye Syndrome

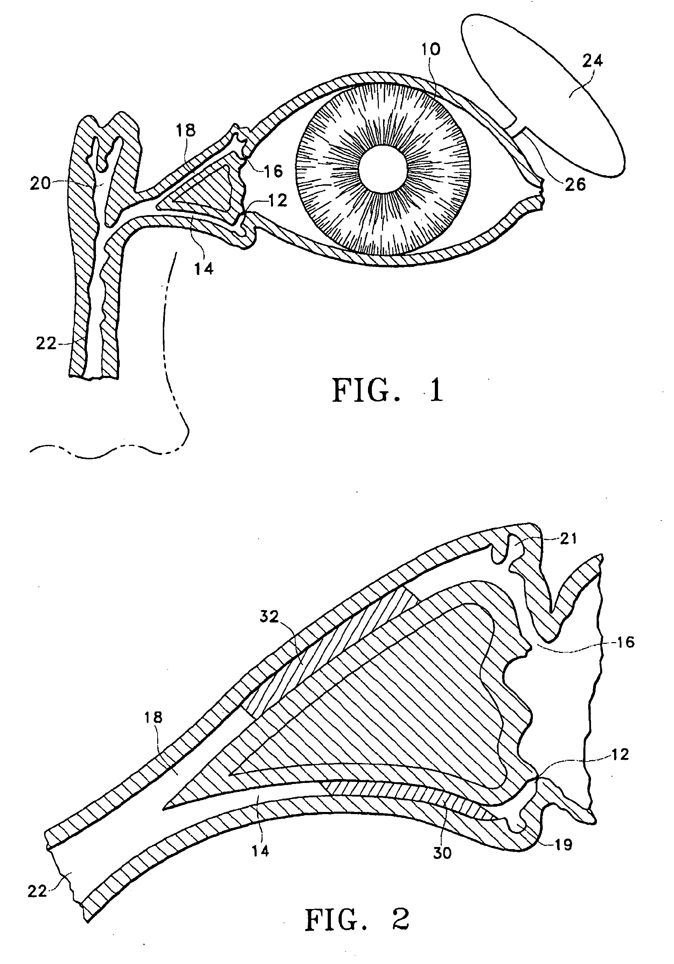

In certain embodiments, the compositions of the present invention can be used to treat dry eye syndrome. FIG. 1 provides an illustration of the lacrimal duct system of a mammalian eye **10.** The system includes a lower punctum **12** connected to a lower lacrimal canaliculus **14,** and an upper punctum **16** connected to an upper lacrimal canaliculus **18.** Canaliculi **14** and **18** are connected to a lacrimal sac **20** and a nasolacrimal duct **22.** A lacrimal gland **24** is connected to eye **10** *via* a lacrimal duct **26.** In general, tears are produced by lacrimal gland **24** and are provided to eye **10** *via* lacrimal duct **26,** and tears are drained from eye **10** via punctum **12** and canaliculus **14,** punctum **16** and canaliculus **18,** and nasolacrimal duct **22.**

As discussed above, the effects of dry eye syndrome can be treated by occluding the canalicular **14, 18** or punctal **12**, **16** channels of the mammalian eye **10** which can reduce or prevent drainage of tear fluid from the eye through the canalicular and punctal channels. For instance, the nozzle of the injecting device can be inserted into through the lower or upper punctal openings and into punctal **12, 16** and/or canalicular **14, 18** channels. The composition can be injected out of the nozzle and into the channels. The injection is continued until the desired amount of blockage is obtained. In some instances, it may be desirable to only block part of the channel *(i.e.,* allow partial flow) or the entire channel. In certain aspects, the composition can be injected into the channel so as to fill the channel and allow the polymeric material to conform to the internal surface walls of the channel being blocked. Thereafter, the nozzle of the injecting device is withdrawn.

FIG. 2 illustrates the lacrimal canaliculi **14** and **18** blocked or occluded by the composition of the present invention **30** and **32,** respectively. Although the composition **30** and **32** as shown in FIG. 2 completely block canaliculi **14** and **18,** the composition can be formulated and/or inserted in a manner that so as to only partially block canaliculi **14** and **18,** if desired. Additionally, the composition **30** and **32** may also be formed to extend into ampullae **19** and **21,** if desired, or any other part of the channels shown in FIGS 1 and 2.

### B. Water-Insoluble Polymer Matrix

Water insoluble polymer matrices of the present invention can be formulated into an injectible paste that can be used to occlude a channel in a subject. In certain aspects, the polymer matrices have a melting point of less than 60 °C. The matrices preferably comprise polymers having an average molecular weight of 400 to 8000 and/or a viscosity of 50 to 8000 cps at 55 °C.

### 1. Bioerodable Polyester Polymers

The matrices of the present invention can include polyester polymers. Non-limiting examples of bioerodable polyester polymers that can be used include poly(ε-caprolactone)s, poly(alkylene glycol adipate)s, such as poly(ethylene glycol adipate), poly(propylene glycol adipate), poly(butylene glycol adipate), and blends and copolymers thereof. Poly(caprolactone) polymers are a preferred polyester polymer and are commercially available from Dow Chemical Company (located in Midland Michigan) and Solvay Chemicals, Inc. (located in Houston, Texas) under the trade names TONE™ Polyol and CAPA™ Polyol, respectively.

Table 1 below includes non-limiting examples of water-insoluble polyester polymer matrices that can be used in the context of the present invention. These polymers are based on polycaprolactone. The physical properties of these polymers and their commercial availability are listed in Table 1:

**Table 1**

| | **PCL-900** | **Tone 310** | | **CAPA 2101a** | **Tone 2221** | **CAPA PL-1000** | **Tone 1231** |
|---|---|---|---|---|---|---|---|
| **Supplier** | Sigma | Dow | | Solvay | Dow | Solvay | Dow |
| **Polyol** | TMP | TMP | | 2-NPG | 2-NPG | none | BDO |
| **MW** | 900 | 900 | | 1000 | 1000 | 1000 | 1250 |
| **Mp/ °C** | Softening point 30 °C | 27-32 L | | 30-40 | 15-40 | 10-20 | 20-45 |
| **Physical form at RT** | paste | paste | | paste | paste | paste | wax |
| **Viscosity/ cps** | 272 @ 55 C | 270 @ 55 C | | 150 @ 60 C | 180 @ 55 C | 150 @ 60 C | 200 @ 55 C |

| | | | | | | | |
|---|---|---|---|---|---|---|---|
| PCL-900 = Tone 310 = Polycaprolactone triol with TMP as triol CAPA 2101A = Tone 2221 - Polycaprolactone diol with NPG as the diol CAPA PL-1000 = Polycaprolactone with mw of 1000 with no polyols Tone 1232 = Polycaprolactone diol with butane diol (BDO) as the diol TMP= Trimethylol propane NPG = Neopantyl glycol BDO = Butene diol PENTA = Pentaerythritol | | | | | | | |

The erosion rates of the polymers in Table 1 were determined by monitoring weight loss after storing in phosphate buffered saline (PBS) at pH 7.4. The weight loss was determined after exposing the polymer to PBS saline at 37 °C. The saline was removed and vial was dried and weight loss was determined. The rate of erosion after 2 weeks and 4 weeks are shown below in Table 2. The pH of the saline solution (originally at pH 7.4) is also reported below.

**Table 2**

| | **PCL-900** | | **Tone 310** | | | **APA2101A** | | **Tone 2221** | | **CAPA PL-1000** | | **Tone 1231** | |
|---|---|---|---|---|---|---|---|---|---|---|---|---|---|
| **Supplier** | Sigma | | Dow | | | Solvay | | Dow | | Solvay | | Dow | |
| **polyol** | TMP | | TMP | | | 2-NPG | | 2-NPG | | none | | | |
| **MW** | 900 | | 900 | | | 1000 | | 1000 | | 1000 | | 1250 | |
| **Mp/°C** | Softening point 30 °C | | 27-32 | | | 30-40 | | 15-40 | | 10-20 | | 20-45 | |
| **Physical form at RT** | Paste | | Paste | | | Paste | | paste | | paste | | wax | |
| **Viscosity/ cps** | 272@55C | | 270 @ 55 C | | | 150@60C | | 180@55C | | 154 @ 60 C | | 200 @ 55C | |
| **Volatile Impurities %(5 hours/80 °C)** | 3.9 | | 2.8 | | | 2.0 | | 1.1 | | 3.5 | | 3.4 | |

| **Erosion** | %* Wt Loss | pH saline | %** Wt Loss | pH saline | | %***Wt Loss | pH saline | %** Wt Loss | pH saline | %** Wt Loss | pH saline | %** Wt Loss | pH saline |
|---|---|---|---|---|---|---|---|---|---|---|---|---|---|
| **@ 2 week** | 5 | NT | 9.8 | 7.01 | | 4.3 | 7.23 | 6.6 | 7.20 | 8.7 | 7.21 | 2.9 | 7.10 |
| **@ 4 week** | 4.5 | 6.87 | 8.9 | 6.87 | | 4.0 | 7.23 | 3.0 | 7.20 | 3.8 | 7.21 | 3.5 | 7.10 |

| | | | | | | | | | | | | | |
|---|---|---|---|---|---|---|---|---|---|---|---|---|---|
| * 1 g polymer in 10 ml PBS at pH 7.4 @ 37 °C ** 1 g polymer in 20 ml PBS at pH 7.4 @ 37 °C *** 0.5 g polymer in 20 ml PBS at pH 7.4 @ 37°C NT = Not Tested | | | | | | | | | | | | | |

In other non-limiting aspects, the polyester polymers of the present invention can be selected from the group consisting of: 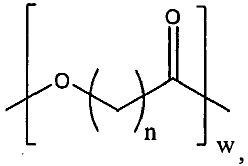 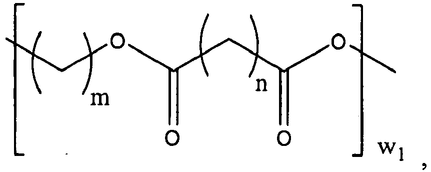 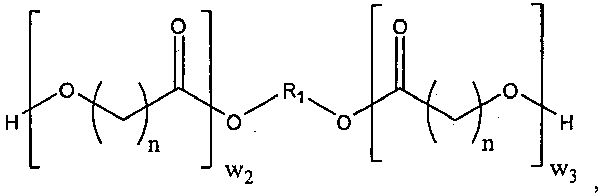 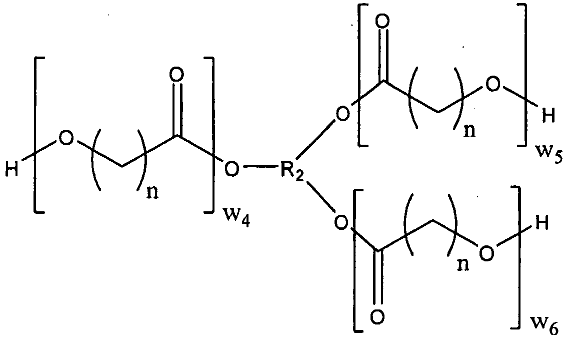 and 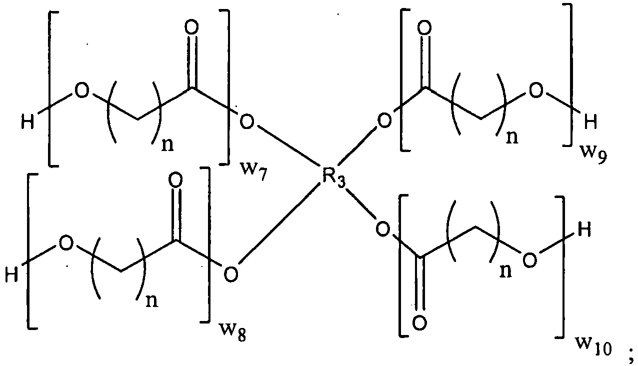 wherein
R₁, R₂ and R₃ are independently selected from the group consisting of alkyl and alkoxyl diols, triols and tetraols of 2 to 8 carbon atoms;
w, w₁ are independently an integer from 4 to 12;
w₂, w₃ are independently an integer from 1 to 12;
w₄, w₅, w₆, w₇*,* w₈, w₉ and w₁₀ are independently an integer from 0 to 12;
n is an integer from 4 to 9; and
m is an integer from 2 to 8.

Non-limiting examples of the alkyl and alkoxyl diols, triols and tetraols of R₁, R₂ and R₃ include butanediol, hexanediol, neopentyl glycol, diethylene glycol, trimethylol propane and pentaerythritol.

The polyester polymer matrices of the present invention can be prepared by a number of different processes known to those of ordinary skill in the art. For instance, polyester polymer matrices can be prepared by heating to reduce viscosity and sterile filtering such as by using an 0.22 µm membrane filter. Further, the polymer matrices can be sterilized by a number of different processes known to those of ordinary skill in the art. For instance, the polymer matrices can be sterilized by dry heat, gamma irradiation, or other common methods of sterilization.

The polyester polymer matrices of the present invention generally comprise a polyester polymer in an amount of at least 50%, preferably at least 70%, and more preferably at least 80%. In some embodiments, the compositions comprise a polyester polymer in an amount of at least 85%. In other embodiments, the compositions of the present invention comprise a polyester polymer in an amount of at least 95%. In yet another embodiment, the compositions comprise a polyester polymer in an amount of at least 99%.

### 2. Fatty Acid Based Polyester Polymers

The matrices of the present invention can include a fatty acid based polymer. Fatty acid based polyesters are currently used in a wide variety of products, such as coatings, adhesives, sealants, elastomers and foams, and have also found use in cosmetic and personal care products. Certain fatty acid based polymers are dimers, and non-limiting examples of such dimers include structures such as:

A-(B-A)ₙ-B-A,

wherein A is an aliphatic hydroxycarboxylic acid ester from 6 to 60 carbon atoms; B is a diacid dimer of unsaturated fatty acids from 8 to 40 carbon atoms; and n is an integer from 0 to 3. In certain embodiments, the aliphatic hydroxycarboxylic acid ester of A has the structure: 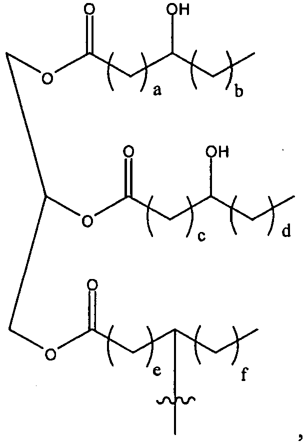 wherein a, b, c, d, e and f are each independently alkyl groups ranging from 1-15 or more linear carbons. In certain embodiments, the diacid dimer of unsaturated fatty acids of B has the structure: 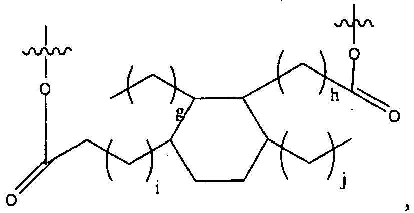 wherein g, h, i and j are each independently alkyl groups ranging from 1-15 or more linear carbons. In certain particular embodiments, A is 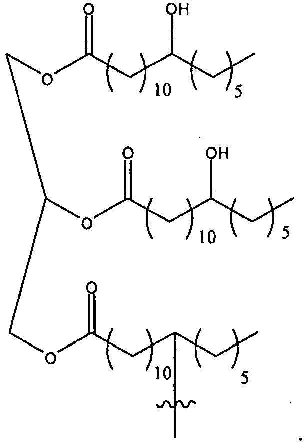 In certain particular embodiments, B is

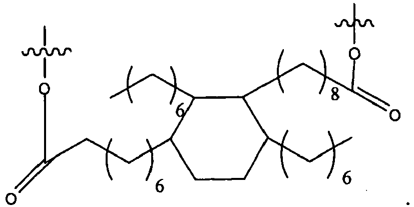

Fatty acid based polymers of the present invention may also be represented by the following structure: 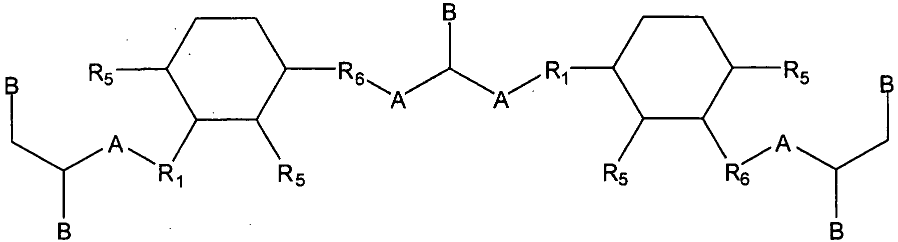 wherein:
A is 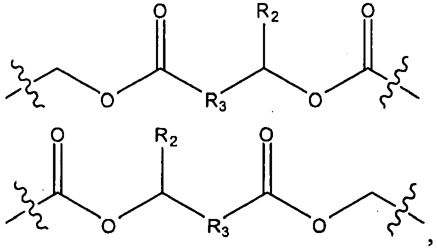 or
B is 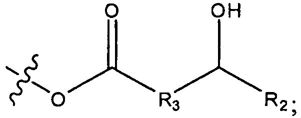 and R₁, R₂, R₃, R₅ and R₆ are each independently alkyl groups ranging from 1-30 or more linear carbons.

Such fatty acid based polyesters are prepared by esterification reaction of diols or polyols such as aliphatic hydroxycarboxylic acids with a fatty acid dimer. An example of a fatty acid-based dimer is the hydrogenated castor oil dimer dilinoleate, which is available from Kokyu Alcohol Kogyo Co., Ltd, (located in Tokyo, Japan) (*e.g.*, RISCOCAST, 3900 and 6800 g/mol molecular weight):

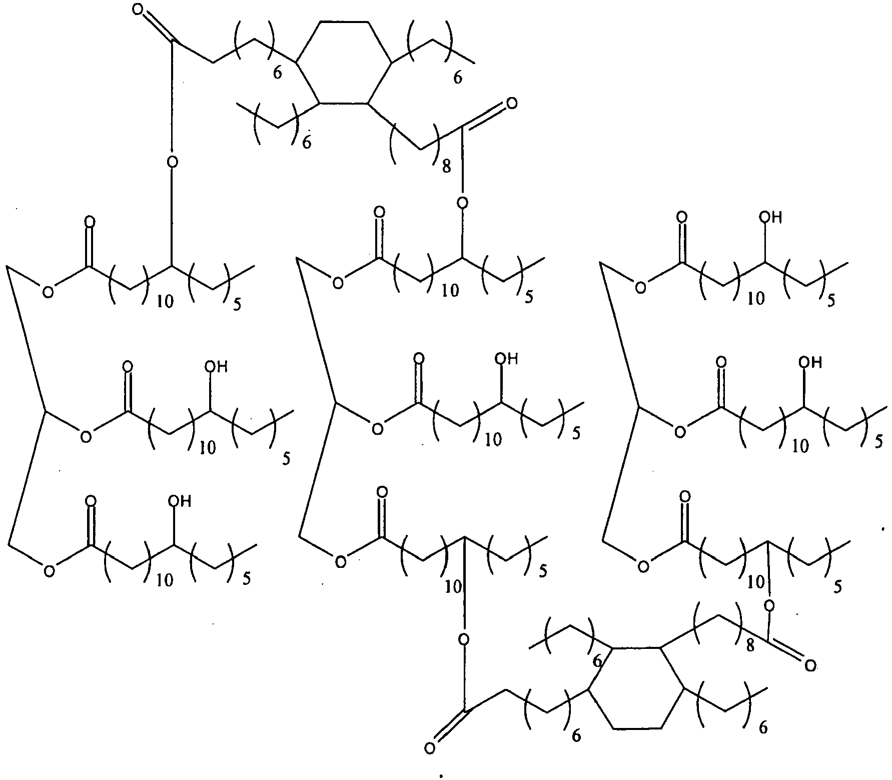

Fatty acid based polymers such as the dimers described above have many advantages over other polymers, such as polyethers and polyurethanes. For example, fatty acid based polymers undergo only slow biodegradation and are relatively stable to heat, oxidation and ultraviolet (UV) light. Further, the hydrophobic environment surrounding the relatively few ester bonds offers both compatibility with organic compounds and relatively high hydrolytic stability when compared to other polyester polyols. The hydrolytic stability of these esters also offers resistance by acids, alkalis and alcohols.

Other features of fatty acid based polymers include good retention of tensile strength and elongation compared to certain polyols and polyethers (*e.g.*, 1,4-butanediol-adipate (BDO-adipate), polypropylene glycol (PPG) and polybutadiene-diol (PB-diol), flexibility with good impact strength and adhesion to low-energy surfaces. For example, fatty acid based polyester polyol dimers sold by Uniqema (located in Gouda, The Netherlands) under the trade name PRIPLAST (1300-3200 g/mol molecular weight range) exhibit these characteristics. PRIPLAST polymers can also easily be converted to polyurethanes that feature characteristics such as good elongation, tensile strength and Shore A hardness. When used in the context of the present invention, fatty acid based polymers such as PRIPLAST and/or RISCOCAST may impart favorable properties as described above.

### C. Active Agents

In certain non-limiting aspects, the water insoluble polymer matrices of the present invention can include an active agent. Active agents include, but are not limited to, any component, compound, or small molecule that can be used to bring about a desired effect. Non-limiting examples of desired effects of the present invention include therapeutic effects. For example, a desired effect can include the diagnosis, cure, mitigation, treatment, or prevention of a disease or condition. An active agent can also affect the structure or function of body part or organ in a subject.

In certain embodiments, the active agent is a hydrophobic drug. A hydrophobic active agent includes an agent that is sparingly soluble in aqueous media (*e.g.*, not completely dissolved in the media at the concentration at which it is administered in an aqueous composition). Thus, depending upon the use and concentration, an active agent may be considered water-insoluble in one situation but not water-insoluble in another situation. However, a person of ordinary skill in the art would recognize that the active agent does not need to be a hydrophobic drug in the context of the present invention.

A preferred class of active agents includes ophthalmic drugs. Non-limiting examples include: anti-glaucoma agents, anti-angiogenesis agents; anti-infective agents; a anti-inflammatory agents; growth factors; immunosuppressant agents; and anti-allergic agents. Anti-glaucoma agents include beta-blockers, such as timolol, betaxolol, levobetaxolol, and carteolol; miotics, such as pilocarpine; carbonic anhydrase inhibitors, such as brinzolamide and dorzolamide; prostaglandins, such as travoprost, bimatoprost, and latanoprost; seretonergics; muscarinics; dopaminergic agonists; and adrenergic agonists, such as apraclonidine and brimonidine. Anti-angiogenesis agents include anecortave acetate (RETAANE™, Alcon™ Laboratories, Inc. of Fort Worth, Tex.) and receptor tyrosine kinase inhibitors. Anti-infective agents include quinolones, such as ciprofloxacin, moxifloxacin, and gatifloxacin, and aminoglycosides, such as tobramycin and gentamicin. Anti-inflammatory agents include non-steroidal and steroidal anti-inflammatory agents, such as suprofen, diclofenac, ketorolac, nepafenac, rimexolone, and tetrahydrocortisol. Growth factors include EGF. Anti-allergic agents include olopatadine and epinastine. The ophthalmic drug may be present in the form of a pharmaceutically acceptable salt, such as timolol maleate, brimonidine tartrate or sodium diclofenac.

In one embodiment, the ophthalmic drug is selected from the group consisting of known classes of ocular hypotensive drugs, such as carbonic anhydrase inhibitors, beta-blockers, prostaglandins, bradykinin agonists, rho-kinase inhibitors, CNP receptor agonists, and guanylate cyclase activators.

Although ophthalmic drugs are a preferred active agent of the present invention, the inventors contemplate that other active agents can be used. The following includes non-limiting examples of these other active agents, and it should be recognized that some these active agents may be generic to or identical to the ophthalmic drugs identified above. A reason for this is that some ophthalmic drugs can be used to treat or prevent other diseases or conditions. Further, it is also possible that some of the following active agents that are not identified in the above section can be used to treat ophthalmic diseases or conditions. Active agents such as nucleic acids, proteins and peptides, hormones and steroids, chemotherapeutics, NSAIDs, vaccine components, analgesics, antibiotics, anti-depressants, etc. are contemplated as being useful in the context of the present invention.

### D. Pharmaceutical Compositions

In certain aspects, an active agent is dispersed throughout the matrix. The phrases "pharmaceutical or pharmacologically acceptable" can include but are not limited to molecular entities and compositions that do not produce an adverse, allergic or other untoward reaction when administered to a subject, such as, for example, a human. The preparation of a pharmaceutical composition is generally known to those of skill in the art. Remington' s Pharmaceutical Sciences, 18th Ed. Mack Printing Company, 1990. Moreover, for animal (*e.g.*, human) administration, it is preferred that the preparations meet sterility, pyrogenicity, general safety and purity standards as required by FDA Office of Biological Standards.

"Therapeutically effective amounts" are those amounts effective to produce beneficial results in the recipient. Such amounts may be initially determined by reviewing the published literature, by conducting *in vitro* tests or by conducting metabolic studies in healthy experimental animals. Before use in a clinical setting, it may be beneficial to conduct confirmatory studies in an animal model, preferably a widely accepted animal model of the particular disease to be treated. Preferred animal models for use in certain embodiments are rodent models, which are preferred because they are economical to use and, particularly, because the results gained are widely accepted as predictive of clinical value.

"Pharmaceutically acceptable carrier" includes any and all solvents, dispersion media, coatings, surfactants, antioxidants, preservatives (*e.g.*, antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (Remington's, 1990).

The actual dosage amount of a composition of the present invention administered to a subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

The compositions containing bioerodable polymer matrix may additionally contain up to 20% water miscible organic liquids that are miscible in above polyester polymers. In some embodiments, the amount of water miscible organic liquid is 10% or less. Examples of miscible organic liquids are propylene glycol and polyethylene glycols. These liquids are also water soluble and thus will modify the drug release profile and erosion rate of polyester polymer matrix in-vivo. Additionally, these organic liquids reduce the viscosity of the drug loaded matrix.

The composition should be stable under the conditions of manufacture and storage, and preserved against the contaminating action of microorganisms, such as bacteria and fungi. It will be appreciated that exotoxin contamination should be kept minimally at a safe level, for example, less that 0.5 ng/mg protein.

A person of ordinary skill will recognize that the compositions of the present invention can include any number of combinations of ingredients (*e.g.*, active agent, polymers, excipients, etc.). It is also contemplated that that the concentrations of these ingredients can vary. In certain non-limiting embodiments, pharmaceutical compositions may comprise, for example, at least about 0.001%, by weight , of an active ingredient. In other embodiments, the active ingredient may comprise from about 0.002% to about 50% of the weight of the compositions, and any range derivable therein. In still other embodiments, the active ingredient may comprise from about 0.5% to about 5% of the compositions. In further embodiments, the concentration of active agent is about 5% to about 30%. In still further embodiments, the concentration of active agent in the device is about 10% to about 20% by weight. A person of ordinary skill in the art would understand that the concentrations can vary depending on the addition, substitution, and/or subtraction of ingredients in a given composition.

### E. Source of Ingredients

The ingredients and components of the compositions of the present invention that are described in the claims and specification can be obtained by any means known to a person of ordinary skill in the art. In a non-limiting embodiment, for example, these ingredients can be isolated by obtaining the source of such compounds, agents, and active ingredients. In many instances, the ingredients are commercially available.

### H. Kits

In further embodiments of the invention, there is a provided a kit. The kit can include, in non-limiting aspects, the pharmaceutical compositions of the present invention and other ingredients described in the claims and specification. Containers of the kits can include a bottle, dispenser, package, compartment, syringe, needle (*e.g.*, gauge of 7, 8, 9, 10, 15, 20, 25, 30, 31, 32, 33, *etc.*) or other types of containers. The container can include indicia on its surface. The indicia, for example, can be a word, a phrase, an abbreviation, a picture, or a symbol.

The containers can dispense a pre-determined amount of the component (*e.g.* compositions of the present invention). The composition can be dispensed in a spray, an aerosol, or in a liquid form or semi-solid form. The containers can have spray, pump, or squeeze mechanisms. The kit includes a syringe for administering the compositions of the present invention.

Where there is more than one component in the kit (they may be packaged together), the kit also will generally contain a second, third or other additional containers into which the additional components may be separately placed. The kits of the present invention also can include a container housing the components in close confinement for commercial sale. Such containers may include injection or blow-molded plastic containers into which the desired bottles, dispensers, or packages are retained.

A kit can also include instructions for employing the kit components as well the use of any other compositions, compounds, agents, active ingredients, or objects not included in the kit. Instructions may include variations that can be implemented. The instructions can include an explanation of how to apply, use, and maintain the products or compositions, for example.

### REFERENCES

U.S. Patent 3,949,750
U.S. Patent 5,283,063
U.S. Patent 5,723,005
U.S. Patent 5,171,270
U.S. Patent 5,469,867
U.S. Patent 5,469,867
U.S. Patent 6,995,186
U.S. Patent Publn. 2003/0055102
U.S. Patent Publn. 2005/0158387

Remington's Pharmaceutical Sciences, 18th Ed. Mack Printing Company, 1990

## Claims

1. An injectable paste composition for use in a therapeutic method for occluding a canalicular or a punctal channel in a subject, comprising:
a water insoluble polymer matrix that comprises a polyester polymer or a fatty acid based polymer, or a mixture of both polymers, wherein the polymer matrix has a melting point of less than 60°C and is paste at room temperature,
wherein said polyester polymer is selected from the group consisting of; 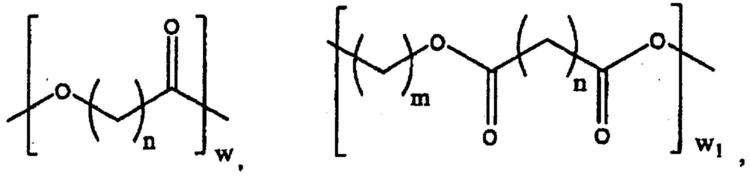 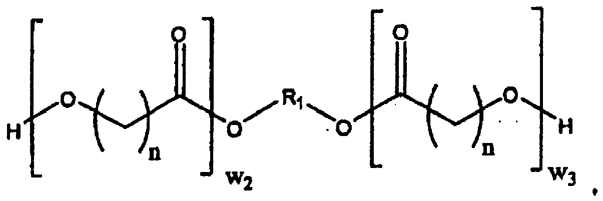 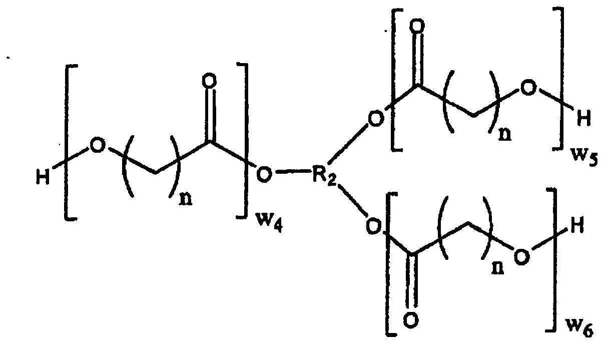 and 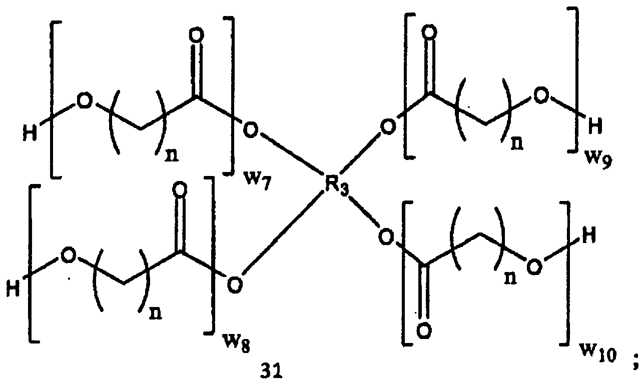
wherein
R₁, R₂ and R₃ are independently selected from the group consisting of alkyl and alkoxyl diols, triols and tetraols of 2 to 8 carbon atoms;
w, w₁ are independently an integer from 4 to 12; w₂, w₃ are independently an integer from 1 to 12;
w₄, w₅, w₆, w₇, w₈, w₉ and w₁₀ are independently an integer from 0 to 12;
n is an integer from 4 to 9; and
m is an integer from 2 to 8;
and wherein said fatty acid based polymer has the following structure:
A-(B-A)ₙ-B-A,
wherein
A is an aliphatic hydroxycarboxylic acid ester from 6 to 60 carbon atoms;
B is a diacid dimer of unsaturated fatty acids from 8 to 40 carbon atoms; and
n is an integer from 0 to 3.

2. The injectable composition of claim 1 for use in the method according to claim 1, wherein the composition comprises up to 50 % (w/w) of a water miscible organic liquid.

3. The injectable composition of claim 1 for use in the method according to claim 1, wherein the polymer matrix comprises a polyester polymer selected from the group consisting of; poly(caprolactone)s; poly(ethylene glycol adipate)s; poly(propylene glycol adipate)s; poly(butylene glycol adipate)s; poly(hydroxybutarate)(s); poly(hydroxyvalerate)(s); and blends and copolymers thereof

4. The injectable composition of claim 3 for use in the method according to claim 1, wherein the polymer matrix comprises a poly(ε-caprolactone) polymer.

5. The injectable composition of claim 1 for use in the method according to claim 1, wherein R₁, R₂ and R₃ are independently selected from the group consisting ofbutanediol, hexanediol, neopentyl glycol, diethylene glycol, trimethylol propane and pentaerythritol.

6. The injectable composition of claim 1 for use in the method according to claim 1, wherein A has the structure: 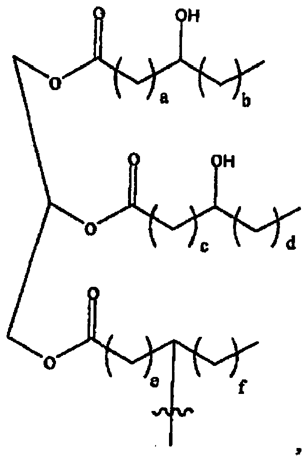 wherein a, b, c, d, e and f are independently alkyl groups ranging from 1-15 or more linear carbons.

7. The injectable composition of claim 1 for use in the method according to claim 1, wherein B has the structure: 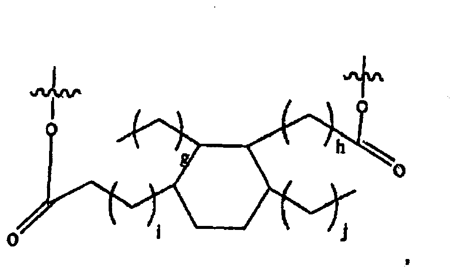 wherein g, h, i and j are independently alkyl groups ranging from 1-15 or more linear carbons.

8. The pharmaceutical composition of claim 1 for use in the method according to claim 1, wherein the polymer matrix comprises a polyester polymer having an average molecular weight of 400 to 8000.

9. The pharmaceutical composition of claim 1, wherein the composition has a viscosity of 50 to 8000 cps at 55°C.

10. The pharmaceutical composition of claim 1 for use in the method according to claim 1, wherein the polymer matrix or the composition is bioerodible.

11. The pharmaceutical composition of any one of claims 1 to 10 for use in the method according to claim 1, wherein an active agent is dispersed within the polymer matrix.

12. A kit comprising an injectable paste composition and a syringe, the composition comprising:
a water insoluble polymer matrix that comprises a polyester polymer or a fatty acid based polymer, or a mixture of both polymers, wherein the polymer matrix has a melting point of less than 60°C and is a paste at room temperature,
wherein said polyester polymer is selected from the group consisting of; 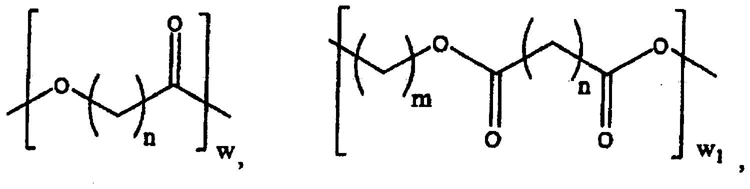 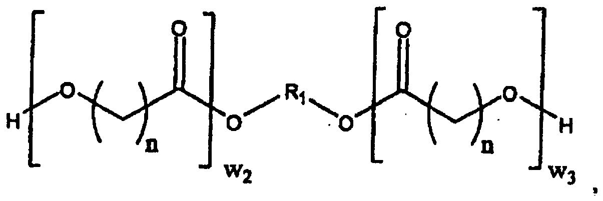 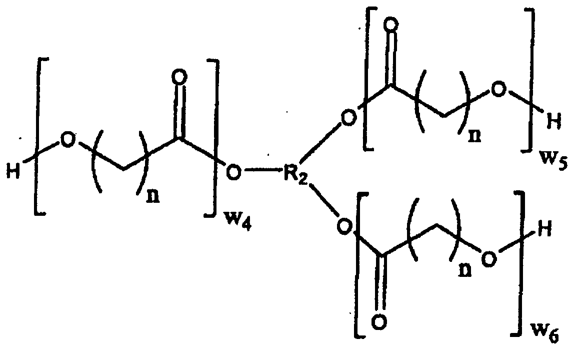 and 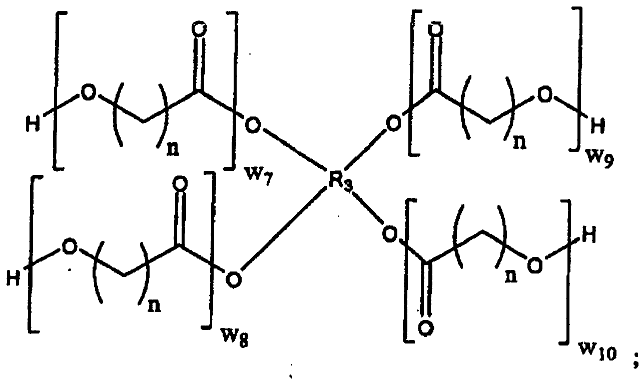
wherein
R₁, R₂ and R₃ are independently selected from the group consisting of alkyl and alkoxyl diols, triols and tetraols of 2 to 8 carbon atoms;
w, w₁ are independently an integer from 4 to 12; w₂, w₃ are independently an integer from 1 to 12;
w₄, w₅, w₆, w₇, w₈, w₉ and w₁₀ are independently an integer from 0 to 12;
n is an integer from 4 to 9; and
m is an integer from 2 to 8;
and wherein said fatty acid based polymer has the following structure:
A-(B-A)ₙ-B-A,
wherein
A is an aliphatic hydroxycarboxylic acid ester from 6 to 60 carbon atoms;
B is a diacid dimer of unsaturated fatty acids from 8 to 40 carbon atoms; and
n is an integer from 0 to 3.

## Patentansprüche

1. Injizierbare pastöse Zusammensetzung zur Anwendung in einem therapeutischen Verfahren zum Verschließen eines Kanalikulus oder eines punktuellen Kanals eines Probanden, aufweisend:
eine wasserunlösliche Polymermatrix, die ein Polyesterpolymer oder eine Fettsäure basierend auf einen Polymer, oder eine Mischung aus beiden Polymeren aufweist, wobei die Polymermatrix einen Schmelzpunkt von unter 60°C hat und bei Raumtemperatur pastös ist,
wobei das besagte Polyesterpolymer ausgewählt ist aus der Gruppe, die besteht aus, 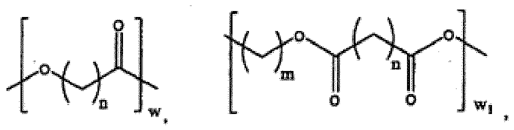 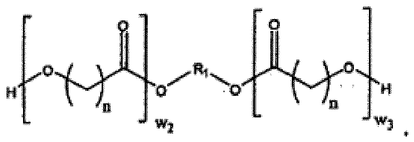 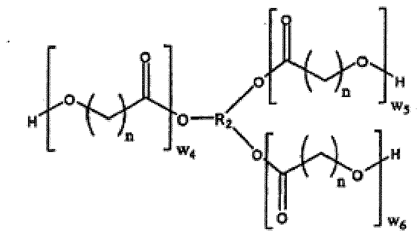 und 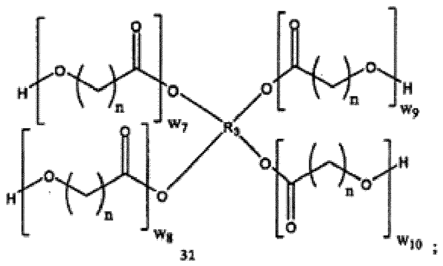
wobei
R₁, R₂ und R₃ unabhängig ausgewählt sind aus der Gruppe bestehend aus Alkyl- und Alkoxyldiolen, -triolen und -tretraolen von 2 bis 8 Kohlenstoffatomen;
w, w₁, unabhängige ganze Zahlen von 4 bis 12 sind; w₂, w₃ unabhängige ganze Zahlen von 1 bis 12 sind;
w₄, w₅, w₆, w₇, w₈, w₉ und w₁₀ unabhängige ganze Zahlen von 0 bis 12 sind;
n eine ganze Zahl von 4 bis 9 ist; und
m eine ganze Zahl von 2 bis 8 ist;
und wobei die auf dem Polymer basierende Fettsäure die folgende Struktur hat:
A-(B-A)ₙ-B-A,
wobei
A ein aliphatischer Hydroxykarboxyl-Säureester von 6 bis 60 Kohlenstoffatomen ist;
B ein Doppelsäuredimmer von ungesättigten Fettsäuren von 8 bis 40 Kohlenstoffatomen ist; und n eine ganze Zahl von 0 bis 3 ist.

2. Injizierbare Zusammensetzung nach Anspruch 1 zur Verwendung in dem Verfahren gemäß Anspruch 1, wobei die Zusammensetzung bis zu 50% (w/w) einer wassermischbaren organischen Flüssigkeit aufweist.

3. Injizierbare Zusammensetzung nach Anspruch 1 zur Verwendung in dem Verfahren gemäß Anspruch 1, wobei die Polymermatrix ein Polyesterpolymer aufweist aus der Gruppe, die besteht aus: Poly(Caprolacton)e; Poly(Ethylen-Glykol-Adipat)e; Poly(Propylen-Glykol-Adipat)e; Poly(Butylen-Glykol-Adipat)e; Poly(Hydroxybutarat)e); Poly(Hydroxyvalerat)e); und Mischungen und Kopolymere daraus.

4. Injizierbare Zusammensetzung nach Anspruch 3 zur Verwendung in dem Verfahren gemäß Anspruch 1, wobei die Polymermatrix ein Poly(ε-Caprolacton)Polymer aufweist.

5. Injizierbare Zusammensetzung nach Anspruch 1 zur Verwendung in dem Verfahren nach Anspruch 1, wobei R₁, R₂ und R₃ unabhängig ausgewählt sind aus der Gruppe, bestehend aus Butandiol, Hexandiol, Neopentylglykol, Diethylen-Glykol, Trimethylolpropan und Pentaerythritol.

6. Injizierbare Zusammensetzung nach Anspruch 1 zur Verwendung in dem Verfahren nach Anspruch 1, wobei A die Struktur hat: 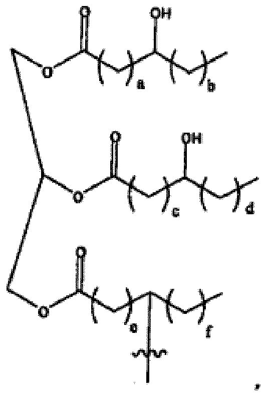 wobei a, b, c, d, e und f unabhängige Alkylgruppen in dem Bereich von 1- 15 oder mehr linearen Kohlenstoffen sind.

7. Injizierbare Zusammensetzung nach Anspruch 1 zur Verwendung in dem Verfahren nach Anspruch 1, wobei B die Struktur hat: 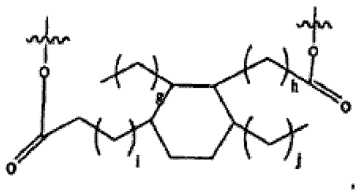 wobei g, h, i und j unabhängige Alkylgruppen in dem Bereich von 1-15 oder mehr linearen Kohlenstoffen sind.

8. Pharmazeutische Zusammensetzung nach Anspruch 1 zur Verwendung in dem Verfahren nach Anspruch 1, wobei die Polymermatrix ein Polyesterpolymer mit einem durchschnittlichen Molekülgewicht von 400 bis 8000 aufweist.

9. Pharmazeutische Zusammensetzung nach Anspruch 1 zur Verwendung in dem Verfahren gemäß Anspruch 1, wobei die Zusammensetzung eine Viskosität von 50 - 8000 cps bei 55°C hat.

10. Pharmazeutische Zusammensetzung nach Anspruch 1 zur Verwendung in dem Verfahren gemäß Anspruch 1, wobei die Polymermatrix oder die Zusammensetzung bio-abbaubar ist.

11. Pharmazeutische Zusammensetzung nach einem der Ansprüche 1 - 10 zur Verwendung in dem Verfahren gemäß Anspruch 1, wobei ein aktives Mittel innerhalb der Polymermatrix verteilt ist.

12. Einrichtung mit einer injizierbaren pastösen Zusammensetzung und einer Spritze, wobei die Zusammensetzung aufweist:
eine wasserunlösliche Polymermatrix, die ein Polyesterpolymer oder eine Fettsäure basierend auf einen Polymer oder eine Mischung aus beiden Polymeren aufweist, wobei die Polymermatrix einen Schmelzpunkt von unter 60°C hat und bei Raumtemperatur pastös ist,
wobei das besagte Polyesterpolymer ausgewählt ist aus der Gruppe, die sich zusammensetzt aus, 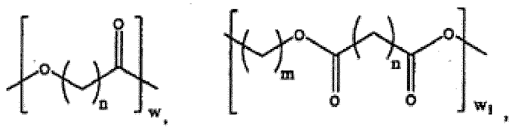 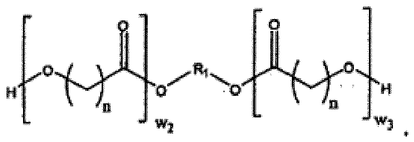 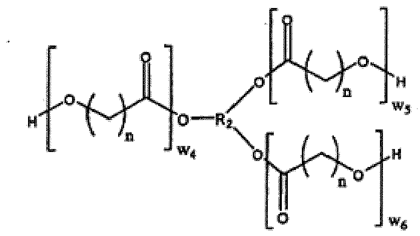 und 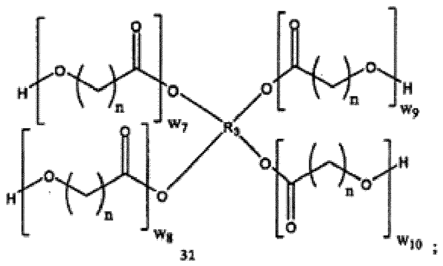 wobei
R₁, R₂ und R₃ unabhängig ausgewählt sind aus der Gruppe bestehend aus Alkyl- und Alkoxyldiolen, -triolen und -tretraolen von 2 bis 8 Kohlenstoffatomen;
w, w₁, unabhängige ganze Zahlen von 4 bis 12 sind; w₂, w₃ unabhängige ganze Zahlen von 1 bis 12 sind;
w₄, w₅, w₆, w₇, w₈, w₉ und w₁₀ unabhängige ganze Zahlen von 0 bis 12 sind;
n eine ganze Zahl von 4 bis 9 ist; und
m eine ganze Zahl von 2 bis 8 ist;
und wobei die auf dem Polymer basierende Fettsäure die folgende Struktur hat:
A-(B-A)ₙ-B-A,
wobei
A ein aliphatischer Hydroxykarboxyl-Säureester von 6 bis 60 Kohlenstoffatomen ist;
B ein Doppelsäuredimmer von ungesättigten Fettsäuren von 8 bis 40 Kohlenstoffatomen ist; und n eine ganze Zahl von 0 bis 3 ist.

## Revendications

1. Composition de pâte injectable pour une utilisation dans un procédé thérapeutique permettant d'occlure un canal canaliculaire ou méatique chez un sujet, comprenant :
une matrice polymère insoluble dans l'eau qui comprend un polymère de polyester ou un polymère à base d'acide gras, ou un mélange des deux polymères, la matrice polymère ayant un point de fusion inférieur à 60 °C et formant une pâte à température ambiante,
dans laquelle ledit polymère de polyester est choisi dans le groupe constitué de : 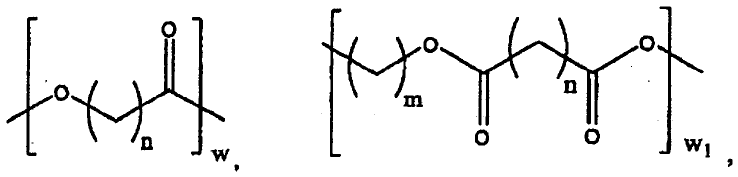 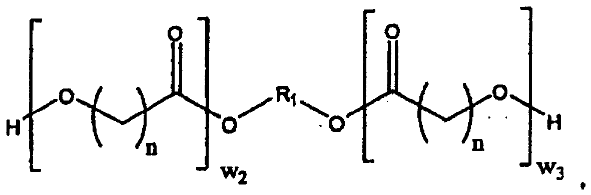 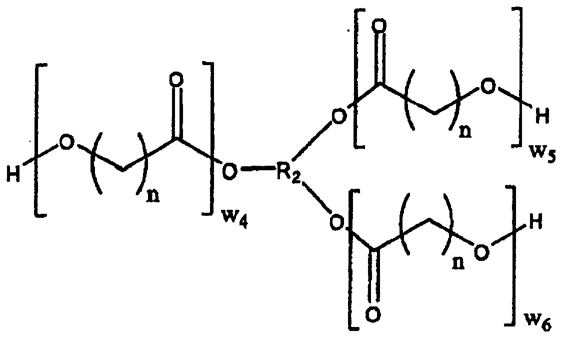 et 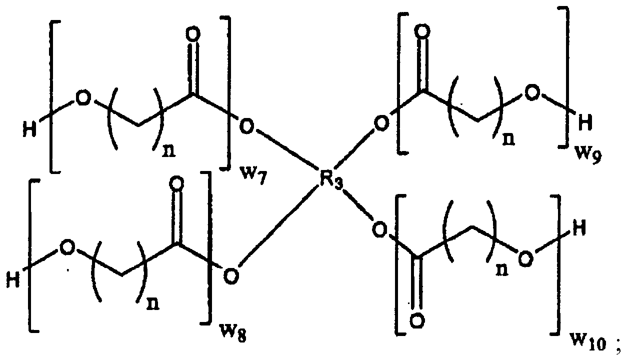 où
R₁, R₂ et R₃ sont indépendamment choisis dans le groupe constitué de diols, triols et tétraols d'alkyle et d'alcoxyle comportant de 2 à 8 atomes de carbone ;
w et w₁ représentent indépendamment un entier de 4 à 12 ; w₂ et w₃ représentent indépendamment un entier de 1 à 12 ;
w₄, w₅, w₆, w₇, w₈, w₉ et w₁₀ représentent indépendamment un entier de 0 à 12 ;
n représente un entier de 4 à 9 ; et
m représente un entier de 2 à 8 ;
et dans laquelle ledit polymère à base d'acide gras a la structure suivante :
A-(B-A)ₙ-B-A,
où
A représente un ester d'acide hydroxycarboxylique aliphatique comportant de 6 à 60 atomes de carbone ;
B représente un dimère de diacide d'acides gras insaturés comportant de 8 à 40 atomes de carbone ; et
n représente un entier de 0 à 3.

2. Composition injectable selon la revendication 1, pour une utilisation dans le procédé selon la revendication 1, la composition comprenant jusqu'à 50 % (p/p) d'un liquide organique miscible à l'eau.

3. Composition injectable selon la revendication 1, pour une utilisation dans le procédé selon la revendication 1, dans laquelle la matrice polymère comprend un polymère de polyester choisi dans le groupe constitué des : poly(caprolactone)s ; poly(adipate d'éthylène glycol)s ; poly(adipate de propylène glycol)s ; poly(adipate de butylène glycol)s ; poly(hydroxybutarate)(s) ; poly(hydroxyvalérate)(s) ; et de mélanges et copolymères de ceux-ci.

4. Composition injectable selon la revendication 3, pour une utilisation dans le procédé selon la revendication 1, dans laquelle la matrice polymère comprend un polymère de poly(ε-caprolactone).

5. Composition injectable selon la revendication 1, pour une utilisation dans le procédé selon la revendication 1, dans laquelle R₁, R₂ et R₃ sont indépendamment choisis dans le groupe constitué du butanediol, de l'hexanediol, du néopentyl glycol, du diéthylène glycol, du triméthylol propane et du pentaérythritol.

6. Composition injectable selon la revendication 1, pour une utilisation dans le procédé selon la revendication 1, dans laquelle A a la structure : 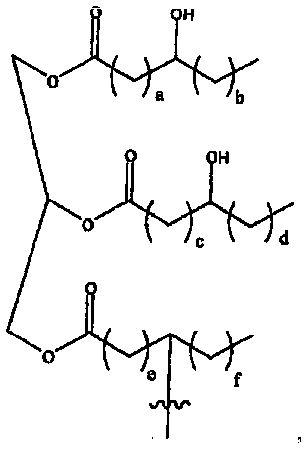 où a, b, c, d, e et f représentent indépendamment des groupes alkyles comportant de 1 à 15 atomes de carbone linéaires ou plus.

7. Composition injectable selon la revendication 1, pour une utilisation dans le procédé selon la revendication 1, dans laquelle B a la structure : 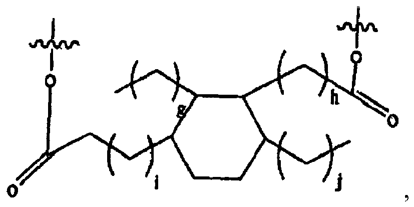 où g, h, i et j représentent indépendamment des groupes alkyles comportant de 1 à 15 atomes de carbone linéaires ou plus.

8. Composition pharmaceutique selon la revendication 1, pour une utilisation dans le procédé selon la revendication 1, dans laquelle la matrice polymère comprend un polymère de polyester ayant un poids moléculaire moyen de 400 à 8 000.

9. Composition pharmaceutique selon la revendication 1, pour une utilisation dans le procédé selon la revendication 1, la composition ayant une viscosité de 50 à 8 000 cps à 55 °C.

10. Composition pharmaceutique selon la revendication 1, pour une utilisation dans le procédé selon la revendication 1, dans laquelle la matrice polymère ou la composition est bioérodable.

11. Composition pharmaceutique selon l'une quelconque des revendications 1 à 10, pour une utilisation dans le procédé selon la revendication 1, dans laquelle un agent actif est dispersé dans la matrice polymère.

12. Kit comprenant une composition de pâte injectable et une seringue, la composition comprenant :
une matrice polymère insoluble dans l'eau qui comprend un polymère de polyester ou un polymère à base d'acide gras, ou un mélange des deux polymères, la matrice polymère ayant un point de fusion inférieur à 60 °C et formant une pâte à température ambiante,
dans laquelle ledit polymère de polyester est choisi dans le groupe constitué de : 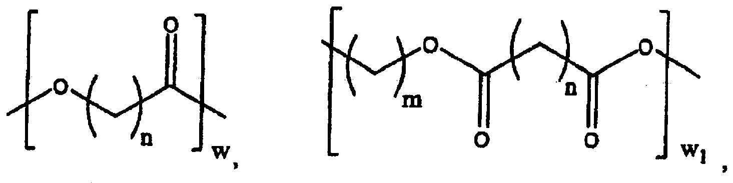 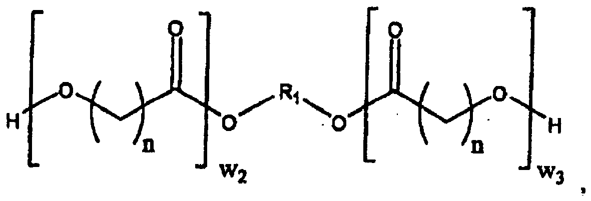 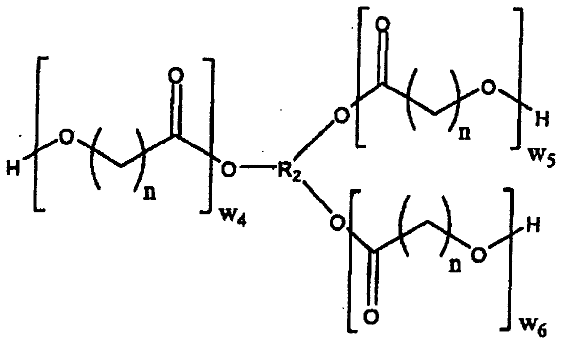 et 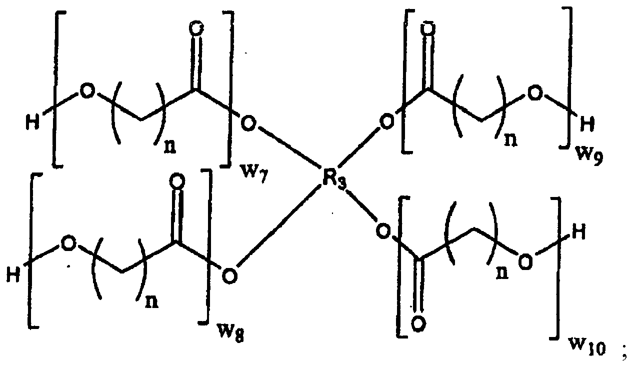
où
R₁, R₂ et R₃ sont indépendamment choisis dans le groupe constitué de diols, triols et tétraols d'alkyle et d'alcoxyle comportant de 2 à 8 atomes de carbone ;
w et w₁ représentent indépendamment un entier de 4 à 12 ; w₂ et w₃ représentent indépendamment un entier de 1 à 12 ;
w₄, w₅, w₆, w₇, w₈, w₉ et w₁₀ représentent indépendamment un entier de 0 à 12 ;
n représente un entier de 4 à 9 ; et
m représente un entier de 2 à 8 ;
et dans laquelle ledit polymère à base d'acide gras a la structure suivante :
A-(B-A)ₙ-B-A,
où
A représente un ester d'acide hydroxycarboxylique aliphatique comportant de 6 à 60 atomes de carbone ;
B représente un dimère de diacide d'acides gras insaturés comportant de 8 à 40 atomes de carbone ; et
n représente un entier de 0 à 3.
